# EUROPÄISCHE PATENTSCHRIFT

(11) **EP 0 739 615 B1**
(45) Veröffentlichungstag und Bekanntmachung des Hinweises auf die Patenterteilung: **14.10.1998**
(21) Anmeldenummer: 96102423.9
(22) Anmeldetag: 17.02.1996
(51) Int. Cl.: A61F 2/68, A61F 2/64, F16F 9/12

(54) **Prothesenbremsgelenk**
Prosthetic brake joint
Articulation prothétique à freinage

(30) Priorität: 31.03.1995 DE 19511890
(43) Veröffentlichungstag der Anmeldung: 30.10.1996
(73) Patentinhaber: Otto Bock Orthopädische Industrie Besitz- und Verwaltungs-Kommanditgesellschaft, 37115 Duderstadt (DE)
(72) Erfinder: Krukenberg, Manfred, D-37115 Duderstadt (DE)
(74) Vertreter: Gramm, Werner, Prof. Dipl.-Ing.

(56) Entgegenhaltungen:
- EP-A- 0 353 336
- WO-A-88/09885
- DE-A- 2 228 391
- DE-U- 9 320 853

## Beschreibung

Die Erfindung betrifft ein Prothesenbremsgelenk, insbesondere Bremskniegelenk für Beinprothesen, mit einem Gelenkoberteil, einem Gelenkunterteil und einer diese beiden Gelenkteile verschwenkbar miteinander verbindenden, drehfest mit dem Gelenkunterteil verbundenen, als Bremsachse ausgebildeten Gelenkachse, die von einer auf ihr als Lager laufenden Bremsbuchse umschlossen ist, die drehfest mit einem Klemmteil verbunden ist, an dem das Gelenkoberteil über eine Schwingachse derart gelenkig gelagert ist, daß das Gelenkoberteil bei Belastung das Klemmteil und dadurch die Bremsbuchse beaufschlagt und so eine Bremswirkung auf die Gelenkachse ausübt.

Eine derartige Ausführungsform läßt sich z.B. der DE-PS 2 228 391 entnehmen. Bei dieser Konstruktion ist das Klemmteil als die Bremsbuchse unmittelbar beaufschlagender Klemmhebel ausgebildet, auf dem sich das Gelenkoberteil abstützt und bei Belastung die Bremsbuchse beaufschlagt und so die Reibung zwischen Bremsbuchse und der als Bremsachse wirkenden Gelenkachse erhöht.

Der Erfindung liegt die Aufgabe zugrunde, die Funktion eines derartigen Prothesenbremsgelenkes zu verbessern.

Diese Aufgabe wird gemäß der Erfindung dadurch gelöst, daß die Bremsbuchse von einer kreisringförmigen Bremskammer umschlossen ist, die mit einem inkompressiblen Medium gefüllt ist und mit einem im Klemmteil integrierten, geschlossenen Hohlraum in flüssigkeitsaustauschender Verbindung steht, in den ein das inkompressible Medium beaufschlagender Druckkolben ragt, an dem sich das Gelenkoberteil abstützt.

Die Ansteuerung des Bremsgelenkes erfolgt somit in Übereinstimmung mit den vorbekannten Ausführungsformen durch Drücken eines Bremsmechanismus, wobei die Reibung in ebenfalls bekannter Weise von einer Bremsbuchse auf eine Bremsachse übertragen wird. Neu ist hingegen die Kraftübergabe vom Ansteuern bis zum Einengen der Bremsbuchse, was erfindungsgemäß mit einem inkompressiblen Medium, wie z.B. Öl, durchgeführt wird.

Bei der erfindungsgemäßen Konstruktion ergibt sich über den gesamten Durchmesser der Bremsbuchse eine gleichmäßige Kraftübertragung. Dadurch wird eine gleichmäßige Anlage der Bremsbuchse an der als Bremsachse fungierenden Gelenkachse erzielt. Hierdurch wird bereits bei niedrigerem Bremsdruck eine höhere Bremsleistung erzielt. Außerdem läßt sich ein verringerter Verschleiß der Bremsbuchse feststellen. Vorteilhaft ist ferner, daß sich Vorspannung und Nachjustierung ausschließlich durch Druckbeaufschlagung des Mediums erzielen lassen.

In konstruktiver Hinsicht ergibt sich als Vorteil die geschlossene Form des Bremsteils und eine hieraus resultierende höhere Verbindungssteifigkeit.

Um z.B. eine axial geschlitzte Bremsbuchse verwenden zu können, ist es zweckmäßig, wenn die Bremsbuchse von einer Dehnhülse umschlossen ist.

Um eine Voreinstellung des Drucks des im Hohlraum befindlichen inkompressiblen Mediums vornehmen zu können, ist es vorteilhaft, wenn in den mit dem inkompressiblen Medium befüllten Hohlraum eine im Klemmteil angeordnete Stellschraube ragt.

Weitere Merkmale der Erfindung sind Gegenstand der Unteransprüche und werden in Verbindung mit weiteren Vorteilen der Erfindung anhand eines Ausführungsbeispiels näher erläutert.

In der Zeichnung ist eine als Beispiel dienende Ausführungsform der Erfindung dargestellt. Es zeigen:
- Figur 1: in Seitenansicht ein Bremskniegelenk in gestreckter Lage;
- Figur 2: das Bremskniegelenk gemäß Figur 1 in gebeugter Stellung;
- Figur 3: einen lotrechten Längsschnitt durch die Darstellung gemäß Figur 1 und
- Figur 4: einen lotrechten Längsschnitt durch die Darstellung gemäß Figur 2.

Das in den Figuren dargestellte Bremskniegelenk umfaßt ein Gelenkoberteil 1, ein Gelenkunterteil 2 sowie eine diese beiden Gelenkteile verschwenkbar miteinander verbindende Gelenkachse 3, die drehfest mit dem Gelenkunterteil 2 verbunden und als Bremsachse ausgebildet ist. Auf der Gelenkachse 3 als Lager läuft eine Bremsbuchse 4, die geschlitzt ausgebildet sein kann und drehfest in einer Dehnhülse 5 verankert ist, die eine sie kreisringförmig umgebende Bremskammer 6 gegenüber der Gelenkachse 3 flüssigkeitsdicht abschließt.

Bremsbuchse 4 und Dehnhülse 5 sind drehfest mit einem Klemmteil 7 verbunden, an dem das Gelenkoberteil 1 über eine Schwingachse 8 gelenkig gelagert ist.

Die Bremskammer 6 steht mit einem nach außen allseitig geschlossenen, im Klemmteil 7 integrierten, mit dem inkompressiblen Medium befüllten Hohlraum 9 in flüssigkeitsaustauschender Verbindung. In diesen Hohlraum 9 ragt ein in einer Längsführung im Klemmteil 7 abdichtend gelagerter Druckkolben 10, der mit seinem inneren Ende das inkompressible Medium beaufschlagt und mit seinem gegenüberliegenden, aus dem Klemmteil 7 herausragenden Ende einen Anschlag für das um die Schwingachse 8 begrenzt verschwenkbare Gelenkoberteil 1.

In den mit dem inkompressiblen Medium befüllten Hohlraum 9 ragt eine im Klemmteil 7 angeordnete Stellschraube 11, durch deren manuelle Verstellung der Druck des inkompressiblen Mediums im Hohlraum 9 voreinstellbar ist.

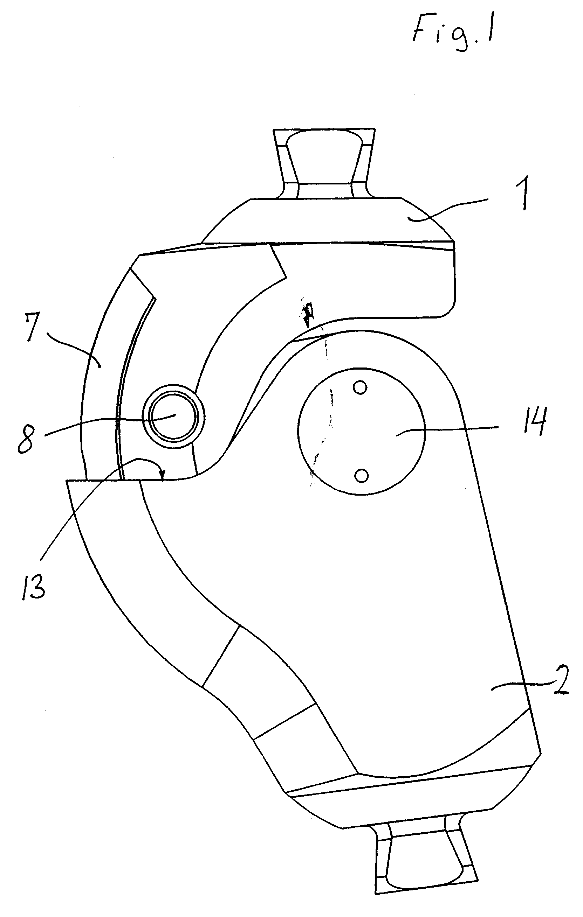

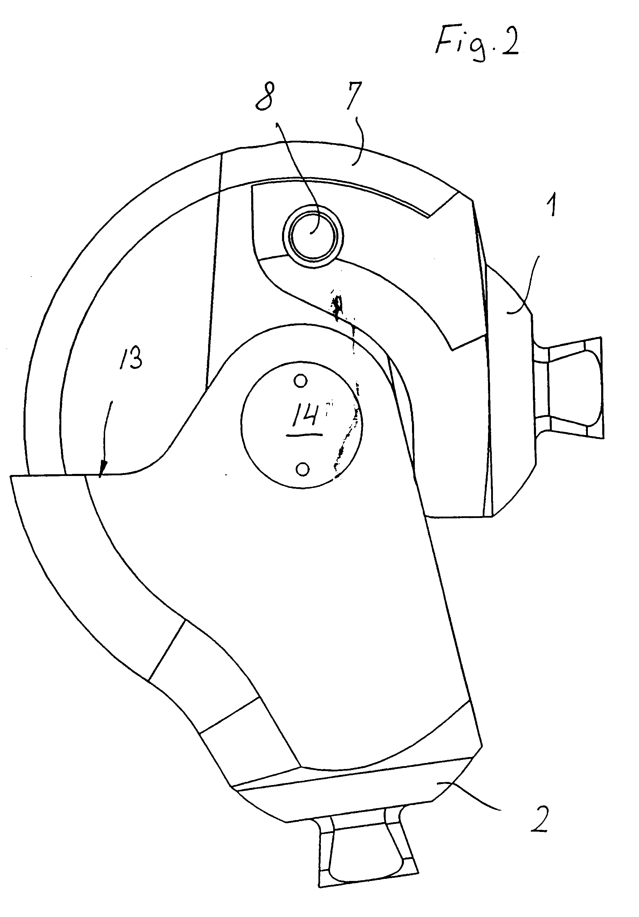

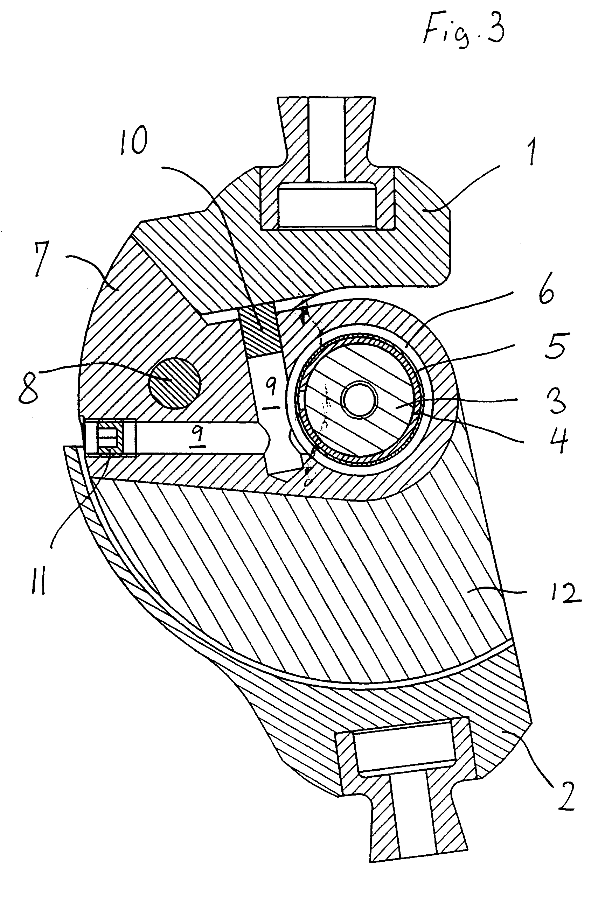

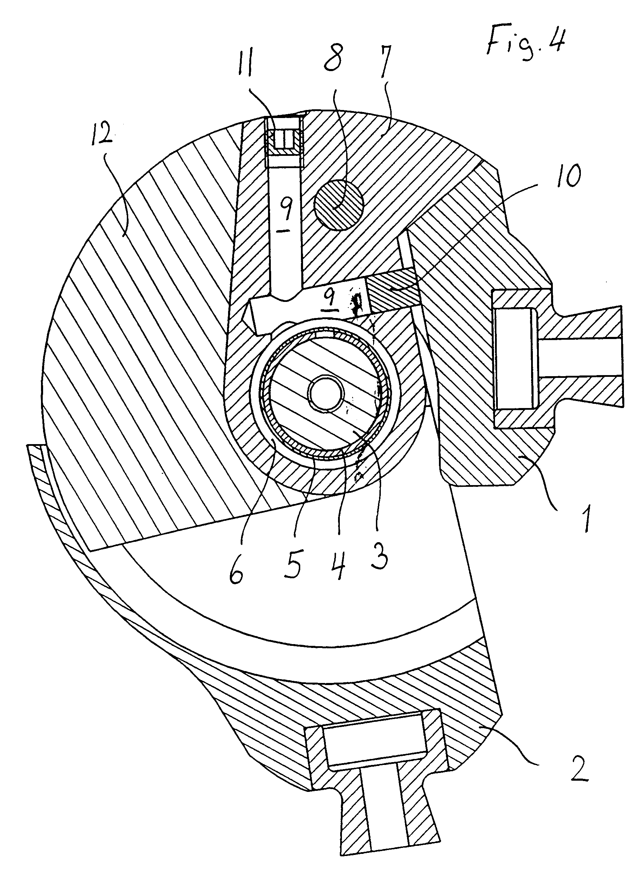

Fest verbunden mit dem Klemmteil 7 ist eine Kniekappe 12, die zur Knieformgebung bei gebeugtem Knie (siehe Figuren 2 und 4) dient. Bei der Bewegung des Gelenkes wird dessen in den Figuren 1 und 3 dargestellte Streckstellung definiert durch einen Streckanschlag 13. Figur 1 läßt überdies eine Achsschraube 14 erkennen, die die Gelenkachse 3 mit dem Gelenkunterteil 2 verbindet.

Unter Belastung verschwenkt das Gelenkoberteil 1 um die drehfest am Klemmteil 7 festgelegte Schwingachse 8 und drückt dadurch auf den Druckkolben 10. Letzterer beaufschlagt infolge dieser belastungsabhängigen Ansteuerung das im Hohlraum 9 befindliche inkompressible Medium mit Druck, nachdem der Ausgangsdruck zuvor mit Hilfe der Stellschraube 11 eingestellt worden war. Durch diese Druckerhöhung des inkompressiblen Mediums wird die Dehnhülse 5 in der Bremskammer 6 auf ihrem gesamten Umfang mit Flüssigkeitsdruck beaufschlagt und drückt dadurch die von ihr konzentrisch umschlossene Bremsbuchse 4 zusammen. Hierdurch wird eine Bremsung gegenüber der Gelenkachse 3 und somit gegenüber einer Relativverschwenkung zwischen Gelenkober- und -unterteil 1, 2 erzeugt.

## Patentansprüche

1. Prothesenbremsgelenk, insbesondere Bremskniegelenk für Beinprothesen, mit einem Gelenkoberteil (1), einem Gelenkunterteil (2) und einer diese beiden Gelenkteile (1, 2) verschwenkbar miteinander verbindenden, drehfest mit dem Gelenkunterteil (2) verbundenen, als Bremsachse ausgebildeten Gelenkachse (3), die von einer auf ihr als Lager laufenden Bremsbuchse (4) umschlossen ist, die drehfest mit einem Klemmteil (7) verbunden ist, an dem das Gelenkoberteil (1) über eine Schwingachse (8) derart gelenkig gelagert ist, daß das Gelenkoberteil (1) bei Belastung das Klemmteil (7) und dadurch die Bremsbuchse (4) beaufschlagt und so eine Bremswirkung auf die Gelenkachse (3) ausübt, **dadurch gekennzeichnet,** daß die Bremsbuchse (4) von einer kreisringförmigen Bremskammer (6) umschlossen ist, die mit einem imkompressiblen Medium gefüllt ist und mit einem im Klemmteil (7) integrierten, geschlossenen Hohlraum (9) in flüssigkeitsaustauschender Verbindung steht, in den ein das inkompressible Medium beaufschlagender Druckkolben (10) ragt, an dem sich das Gelenkoberteil (1) abstützt.

2. Prothesenbremsgelenk nach Anspruch 1, **dadurch gekennzeichnet,** daß die Bremsbuchse (4) von einer Dehnhülse (5) umschlossen ist.

3. Prothesenbremsgelenk nach Anspruch 2, **dadurch gekennzeichnet,** daß die Dehnhülse (5) drehfest mit dem Klemmteil (7) verbunden ist.

4. Prothesenbremsgelenk nach Anspruch 2 oder 3, **dadurch gekennzeichnet,** daß die Bremsbuchse (4) drehfest in der Dehnhülse (5) verankert ist.

5. Prothesenbremsgelenk nach einem der vorhergehenden Ansprüche, **dadurch gekennzeichnet,** daß in den mit dem inkompressiblen Medium befüllten Hohlraum (9) eine im Klemmteil (7) angeordnete Stellschraube (11) ragt.

## Claims

1. Prosthetic brake joint, in particular knee brake joint for leg prostheses, comprising an upper joint section (1), a lower joint section (2) and a joint spindle (3) designed as a brake spindle connecting said two joint sections (1, 2) with one another so as to be capable of swivel motion and connected to the lower section (2) in non-rotating manner, said joint spindle (3) being surrounded by a brake bush (4) moving thereon as a bearing, said brake bush (4) being connected to a clamping section (7) in non-rotating manner, said upper joint section (1) being mounted in articulated manner on said clamping section (7) via a swivel spindle (8) so as to ensure that when subject to loading the upper joint section (1) exerts a force on said clamping section (7) and hence on the brake bush (4) thus applying a braking force to the joint spindle (3), characterised in that the brake bush (4) is surrounded by an annular brake chamber (6) filled with an incompressible medium and communicates in fluid-exchanging manner with an enclosed cavity (9) integrated within the clamping section (7), whereby a plunger (10) acting on the incompressible medium and supporting the upper joint section (1) projects into said cavity (9).

2. Prosthetic brake joint according to Claim 1, characterised in that the brake bush (4) is surrounded by an expanding sleeve (5).

3. Prosthetic brake joint according to Claim 2, characterised in that the expanding sleeve (5) is connected in non-rotating manner to the clamping section (7).

4. Prosthetic brake joint according to Claim 2 or Claim 3, characterised in that the brake bush (4) is anchored in non-rotating manner within the expanding sleeve (5).

5. Prosthetic brake joint according to one of the preceeding claims, characterised in that a set screw (11) arranged within the clamping section (7) projects into the cavity (9) filled with the incompressible medium.

## Revendications

1. Articulation prothétique à freinage, notamment articulation du genou à freinage pour prothèse de la jambe, comprenant une partie supérieure d'articulation (1), une partie inférieure d'articulation (2), et un axe d'articulation (3) formant axe de freinage, reliant l'une à l'autre, de manière pivotante, ces deux parties d'articulation (1, 2), lié en rotation à la partie inférieure d'articulation (2), axe qui est entouré par une bague de freinage (4) qui circule sur lui en tant que palier, la bague étant liée en rotation à une pièce de serrage (7) sur laquelle la partie supérieure d'articulation (1) est supportée de façon articulée au moyen d'un axe d'oscillation (8), de telle manière que sous charge la partie supérieure d'articulation (1) agit sur la pièce de serrage (7) et par suite sur la bague de freinage (4), et exerce ainsi une action de freinage sur l'axe d'articulation (3), caractérisée en ce que la bague de freinage (4) est entourée d'une chambre de freinage (6) annulaire circulaire, remplie d'un milieu incompressible et communiquant par échange de fluide avec une cavité fermée (9) intégrée dans la pièce de serrage (7), cavité dans laquelle plonge un piston presseur (10) agissant sur le milieu incompressible, la partie supérieure d'articulation (1) s'appuyant sur le piston presseur (10).

2. Articulation prothétique à freinage selon la revendication 1, caractérisée en ce que la bague de freinage (4) est entourée d'un manchon extensible (5).

3. Articulation prothétique à freinage selon la revendication 2, caractérisée en ce que le manchon extensible (5) est lié en rotation avec la pièce de serrage (7).

4. Articulation prothétique à freinage selon la revendication 2 ou 3, caractérisée en ce que la bague de freinage (4) est ancrée dans le manchon extensible (5), sans possibilité de rotation entre eux.

5. Articulation prothétique à freinage selon l'une quelconque des revendications précédentes, caractérisée en ce qu'une vis de réglage (11) montée dans la pièce de serrage (7) plonge dans la cavité (9) remplie avec le milieu incompressible.
